# EUROPÄISCHE PATENTSCHRIFT

(11) **EP 1 858 834 B1**
(45) Veröffentlichungstag und Bekanntmachung des Hinweises auf die Patenterteilung: **08.10.2008**
(21) Anmeldenummer: 06706995.5
(22) Anmeldetag: 16.02.2006
(51) Int. Cl.: C07C 59/305, C09K 8/52

(54) **BIOLOGISCH ABBAUBARE GASHYDRATINHIBITOREN**
BIOLOGICALLY DEGRADABLE GAS HYDRATE INHIBITORS
INHIBITEURS D'HYDRATES DE GAZ BIODEGRADABLES

(30) Priorität: 01.03.2005 DE 102005009134
(43) Veröffentlichungstag der Anmeldung: 28.11.2007
(73) Patentinhaber: Clariant Produkte (Deutschland) GmbH, 65929 Frankfurt am Main (DE)
(72) Erfinder: LEINWEBER, Dirk, 65779 Kelkheim (DE); FEUSTEL, Michael, 55278 Köngernheim (DE)
(74) Vertreter: Mikulecky, Klaus
(86) Internationale Anmeldenummer: PCT/EP2006/001394
(87) Internationale Veröffentlichungsnummer: WO 2006/092208

(56) Entgegenhaltungen:
- US-A- 5 244 878
- US-A- 5 432 292
- US-A- 5 879 561
- US-A1- 2004 164 278

## Beschreibung

Die vorliegende Erfindung betrifft Tri- und Tetracarbonsäureester von Aminopolyethem, ihre Herstellung und Verwendung sowie ein Verfahren zur Inhibierung von Keimbildung, Wachstum und/oder Agglomeration von Gashydraten, indem einem zur Hydratbildung neigenden, aus Wasser, Gas und ggf. Kondensat bestehenden Mehrphasengemisch oder einer zur Gashydratbildung neigenden Bohrspülflüssigkeit eine wirksame Menge eines Inhibitors zugegeben wird, der substituierte Polyester enthält.

Gashydrate sind kristalline Einschlussverbindungen von Gasmolekülen in Wasser, die sich unter bestimmten Temperatur- und Druckverhältnissen (niedrige Temperatur und hoher Druck) bilden. Hierbei bilden die Wassermoleküle Käfigstrukturen um die entsprechenden Gasmoleküle aus. Das aus den Wassermolekülen gebildete Gittergerüst ist thermodynamisch instabil und wird erst durch die Einbindung von Gastmolekülen stabilisiert. Diese eisähnlichen Verbindungen können in Abhängigkeit von Druck und Gaszusammensetzung auch über den Gefrierpunkt von Wasser (bis über 25°C) hinaus existieren.

In der Erdöl- und Erdgasindustrie sind insbesondere die Gashydrate von großer Bedeutung, die sich aus Wasser und den Erdgasbestandteilen Methan, Ethan, Propan, Isobutan, n-Butan, Stickstoff, Kohlendioxid und Schwefelwasserstoff bilden. Insbesondere in der heutigen Erdgasförderung stellt die Existenz dieser Gashydrate ein großes Problem dar, besonders dann, wenn Nassgas oder Mehrphasengemische aus Wasser, Gas und Alkangemischen unter hohem Druck niedrigen Temperaturen ausgesetzt werden. Hier führt die Bildung der Gashydrate aufgrund ihrer Unlöslichkeit und kristallinen Struktur zur Blockierung verschiedenster Fördereinrichtungen, wie Pipelines, Ventilen oder Produktionseinrichtungen, in denen über längere Strecken bei niedrigeren Temperaturen Nassgas oder Mehrphasengemische transportiert werden, wie dies speziell in kälteren Regionen der Erde oder auf dem Meeresboden vorkommt.

Außerdem kann die Gashydratbildung auch beim Bohrvorgang zur Erschließung neuer Gas- oder Erdöllagerstätten bei entsprechenden Druck-und Temperaturverhältnissen zu Problemen führen, indem sich in den Bohrspülflüssigkeiten Gashydrate bilden.

Um solche Probleme zu vermeiden, kann die Gashydratbildung in Gaspipelines, beim Transport von Mehrphasengemischen oder in Bohrspülflüssigkeiten durch Einsatz von größeren Mengen (mehr als 10 Gew.-% bezüglich des Gewichts der Wasserphase) niederen Alkoholen, wie Methanol, Glykol, oder Diethylenglykol unterdrückt werden. Der Zusatz dieser Additive bewirkt, dass die thermodynamische Grenze der Gashydratbildung zu niedrigeren Temperaturen und höheren Drücken hin verlagert wird (thermodynamische Inhibierung). Durch den Zusatz dieser thermodynamischen Inhibitoren werden allerdings größere Sicherheitsprobleme (Flammpunkt und Toxizität der Alkohole), logistische Probleme (große Lagertanks, Recycling dieser Lösungsmittel) und dementsprechend hohe Kosten, speziell in der offshore-Förderung, verursacht.

Heute versucht man deshalb, thermodynamische Inhibitoren zu ersetzen, indem man bei Temperatur- und Druckbereichen, in denen sich Gashydrate bilden können, Additive in Mengen < 2 % zusetzt, die die Gashydratbildung entweder zeitlich hinauszögern (kinetische Inhibitoren) oder die Gashydratagglomerate klein und damit pumpbar halten, so dass diese durch die Pipeline transportiert werden können (sog. Agglomerat-Inhibitoren oder Antiagglomerants). Die dabei eingesetzten Inhibitoren behindern entweder die Keimbildung und/oder das Wachstum der Gashydratpartikel, oder modifizieren das Hydratwachstum derart, dass kleinere Hydratpartikel resultieren.

Als Gashydratinhibitoren wurden in der Patentliteratur neben den bekannten thermodynamischen Inhibitoren eine Vielzahl monomerer als auch polymerer Substanzklassen beschrieben, die kinetische Inhibitoren oder Antiagglomerants darstellen. Von besonderer Bedeutung sind hierbei Polymere mit Kohlenstoff-Backbone, die in den Seitengruppen sowohl cyclische (Pyrrolidon- oder Caprolactamreste) als auch acyclische Amidstrukturen enthalten.

So wird in WO-94/12761 ein Verfahren zur kinetischen Inhibierung der Gashydratbildung durch die Verwendung von Polyvinyllactamen mit einem Molekulargewicht von M_{w} > 40.000 D, und in WO-93/25798 wird ein solches Verfahren unter Verwendung von Polymeren und/oder Copolymeren des Vinylpyrrolidons mit einem Molekulargewicht von M_{w} > 5.000 bis 40.000 D offenbart.

EP-A-0 896 123 offenbart Gashydratinhibitoren, die Copolymere aus alkoxylierter Methacrylsäure ohne Alkyl-Endverschluss und cyclischen N-Vinylverbindungen enthalten können.

EP-A-1 048 892 beschreibt die Verwendung von Additiven zur Fließverbesserung von wasserhaltigem Petroleum, wobei dieses Polyvinylalkohol oder teilverseiftes Polyvinylacetat als Keimbildner für Gashydrate in Verbindung mit geeigneten Dispergatoren enthalten kann. Über den Polyvinylalkohol oder das teilverseifte Polyvinylacetat macht das Dokument keine weitere Feststellung, außer dass deren Molekulargewicht unter 50.000 g/mol liegen soll.

In US-5 244 878 wird ein Verfahren zum Verzögern der Bildung oder Vermindern der Bildungstendenz von Gashydraten beschrieben. Hierzu werden Polyole verwendet, die mit Fettsäuren oder Alkenylbernsteinsäureanhydriden verestert werden. Die hergestellten Verbindungen weisen keine Aminosäurefunktionen auf, die mit Clathraten (Käfigmolekülen) interagieren könnten.

Die beschriebenen Additive besitzen nur eingeschränkte Wirksamkeit als kinetische Gashydratinhibitoren und/oder Antiagglomerants, müssen mit Co-Additiven verwendet werden, oder sind nicht in ausreichender Menge oder nur zu hohen Preisen erhältlich.

Um Gashydratinhibitoren auch bei stärkerer Unterkühlung als derzeit möglich, d.h. weiter innerhalb der Hydratregion einsetzen zu können, bedarf es einer weiteren Wirkungssteigerung im Vergleich zu den Hydratinhibitoren des Standes der Technik. Zusätzlich sind in Bezug auf ihre biologische Abbaubarkeit und Toxizität verbesserte Produkte erwünscht.

Aufgabe der vorliegenden Erfindung war es also, verbesserte Additive zu finden, die sowohl die Bildung von Gashydraten verlangsamen (kinetische Inhibitoren) als auch Gashydratagglomerate klein und pumpbar halten (Antiagglomerants), um so ein breites Anwendungsspektrum mit hohem Wirkpotential zu gewährleisten. Des Weiteren sollten sie in der Lage sein, die derzeit verwendeten thermodynamischen Inhibitoren (Methanol und Glykole), die beträchtliche Sicherheitsprobleme und Logistikprobleme verursachen, zu ersetzen.

Wie nun überraschenderweise gefunden wurde, eignen sich sowohl wasserlösliche als auch öllösliche Tri- und Tetracarbonsäureester von Aminopolyethern als Gashydratinhibitoren. Diese Ester können je nach Struktur sowohl die Keimbildung und das Wachstum von Gashydraten verzögern (kinetische Gashydratinhibitoren) als auch die Agglomeration von Gashydraten unterdrücken (Antiagglomerants).

Gegenstand der Erfindung sind daher Tri- und Tetracarbonsäureester der Formel (1) 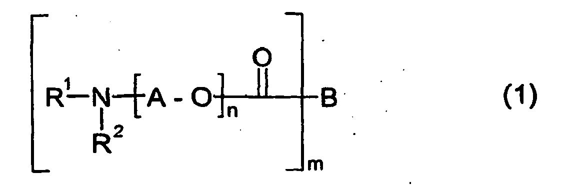 worin
- R¹, R²: unabhängig voneinander C₁- bis C₂₂-Alkyl, C₂- bis C₂₂-Alkenyl, C₆- bis C₃₀-Aryl- oder C₇- bis C₃₀-Alkylaryl, und,
- A: eine C₂- bis C₄-Alkylengruppe,
- B: eine beliebig substituierte organische Gruppe mit 3-100 Kohlenstoffatomen,
- n: eine Zahl von 1 bis 30
- m: 3 oder 4
bedeuten, wobei die Struktureinheiten -NR¹-R² teilweise oder vollständig quatemiert sein können.

Ein weiterer Gegenstand der Erfindung ist ein Verfahren zur Inhibierung der Bildung von Gashydraten, indem einer mit einer gasförmigen, flüssigen oder festen organischen Phase in Verbindung stehenden wässrigen Phase, in der die Gashydratbildung verhindert werden soll, Ester wie vorstehend definiert in Mengen von 0,01 bis 2 Gew.-% zugesetzt werden.

Ein weiterer Gegenstand der Erfindung ist die Verwendung der erfindungsgemäßen Tri- und Tetracarbonsäureester in Mengen von 0,01 bis 2 Gew.-% zur Verhinderung der Bildung von Gashydraten in wässrigen Phasen, die mit einer gasförmigen, flüssigen oder festen organischen Phase in Verbindung stehen.

A steht vorzugsweise für einen Alkylenrest mit 2 oder 3 Kohlenstoffatomen.

R¹ und R² stehen unabhängig voneinander vorzugsweise für C₃- bis C₈-Alkylreste.

n steht vorzugsweise für eine ganze Zahl zwischen 2 und 10, insbesondere zwischen 3 und 6.

B steht vorzugsweise für einen organischen Rest mit 3 bis 10 Kohlenstoffatomen, insbesondere mit 3 bis 6 Kohlenstoffatomen. Vorzugsweise umfasst B Hydroxylgruppen. Besonders bevorzugt umfasst B aliphatische Reste, die gesättigt oder ungesättigt sein können, und 3 bis 6 Kohlenstoffatome enthalten, oder aromatische Reste mit 6 bis 10 Kohlenstoffatomen.

Die erfindungsgemäßen Tri- und Tetracarbonsäureester sind nach literaturbekannten Verfahren durch Veresterung von Tri- oder Tetracarbonsäuren der Formel B(COOH)ₘ mit alkoxylierten Dialkylamine der Formel R¹R²N-(A-O)ₙ-H herstellbar.

Erfindungsgemäß kann auch eine teilweise oder vollständige Quaternierung der Struktureinheiten -NR¹R² der hergestellten Tri- und Tetracarbonsäureester mit gebräuchlichen Alkylierungsmitteln (Alkyl-, Arylhalogenide oder Alkylsulfate) wie beispielsweise Methylchlorid, Benzylchlorid, Monochloressigsäure-Natriumsalz oder Dimethylsulfat erfolgen.

Die Verbindungen der Formel (1) basieren in bevorzugter Ausführungsform auf folgenden Tri- und Tetracarbonsäuren B(COOH)ₘ bzw. deren Anhydriden: Citronensäure, Pyrocitronensäure, Trimellitsäure, Pyromellitsäure, 1,2,3-Benzoltricarbonsäure, 1,3,5-Benzoltricarbonsäure, Butan-1,2,3,4-tetracarbonsäure, Trimellitsäureanhydrid, Pyromellitsäureanhydrid.

Basis der verwendeten alkoxylierten Dialkylamine sind Dialkylamine mit C₁- bis C₂₂-Alkylresten oder C₂- bis C₂₂-Alkenylresten, bevorzugt C₃- bis C₈-Dialkylamine. Geeignete Dialkylamine sind beispielsweise Di-n-butylamin, Diisobutylamin, Dipentylamin, Dihexylamin, Dioctylamin, Dicyclopentylamin, Dicyclohexylamin, Diphenylamin, Dibenzylamin.

Die Alkylamine werden im Allgemeinen mit Ethylenoxid, Propylenoxid, Butylenoxid oder Mischungen verschiedener solcher Alkylenoxide umgesetzt, wobei Ethylenoxid oder Mischungen aus Ethylenoxid und Propylenoxid bevorzugt sind. Bezogen auf die Alkylamine werden 1 bis 30 Mol Alkylenoxid beaufschlagt, bevorzugt 2 bis 10 Mol, besonders bevorzugt 3 bis 6 Mol.

Die Alkoxylierung erfolgt in Substanz, kann aber auch in Lösung durchgeführt werden. Geeignete Lösemittel für die Alkoxylierung sind inerte Ether wie Dioxan, Tetrahydrofuran, Glyme, Diglyme und MPEGs.

Im Allgemeinen wird die Alkoxylierung im ersten Reaktionsschritt unkatalysiert bis auf > 95 Gew.-% tert.-Stickstoff durchgeführt. Höheralkoxylierung erfolgt nach Zugabe basischer Verbindungen als Katalysatoren. Als basische Verbindungen können Erdalkali-/Alkalimetallhydroxide oder -alkoholate (Natriummethylat, Natriumethylat, Kalium-tert.-butylat) verwendet werden, bevorzugt sind aber Alkalimetallhydroxide, besonders Natriumhydroxid oder Kaliumhydroxid.

Die Herstellung von Tri- und Tetracarbonsäureestem ist im Stand der Technik bekannt und erfolgt durch unkatalysierte oder sauer katalysierte Kondensation der jeweiligen Tri- oder Tetracarbonsäure mit dem entsprechenden alkoxylierten Dialkylamin. Die Reaktionstemperatur liegt im Allgemeinen zwischen 100 und 250°C, vorzugsweise bei 120 bis 150°C. Die Reaktion kann bei Atmosphärendruck oder vermindertern Druck durchgeführt werden. Als katalysierende Säuren sind beispielsweise HCl, H₂SO₄, Sulfonsäuren, H₃PO₄ oder saure Ionentauscher zu nennen, die in Mengen von 0,1 bis 5 Gew.-%, bezogen auf das Gewicht des Reaktionsgemisches verwendet werden. Die Kondensation nimmt im Allgemeinen 3 bis 10 Stunden in Anspruch.

Das zahlenmittlere Molekulargewicht der erfindungsgemäßen Tri- und Tetracarbonsäureester liegt vorzugsweise zwischen 500 und 50.000 g/mol, besonders bevorzugt zwischen 1000 bis 10.000 g/mol.

Die Tri- und Tetracarbonsäureester können alleine oder in Kombination mit anderen bekannten Gashydratinhibitoren eingesetzt werden. Im Allgemeinen wird man dem zur Hydratbildung neigenden System soviel des erfindungsgemäßen Gashydratinhibitors zusetzen, dass man unter den gegebenen Druck- und Temperaturbedingungen eine ausreichende Inhibierung erhält. Die erfindungsgemäßen Gashydratinhibitoren werden im Allgemeinen in Mengen zwischen 0,01 und 2 Gew.-% (bezogen auf das Gewicht der wässrigen Phase), entsprechend 100 - 20.000 ppm, vorzugsweise 0,02 bis 1 Gew.-% verwendet. Werden die erfindungsgemäßen Gashydratinhibitoren in Mischung mit anderen Gashydratinhibitoren verwendet, so beträgt die Konzentration der Mischung 0,01 bis 2 bzw. 0,02 bis 1 Gew.-% in der wässrigen Phase.

Die Tri- und Tetracarbonsäureester werden vorzugsweise zur Anwendung als Gashydratinhibitoren in wassermischbaren alkoholischen Lösungsmitteln wie z.B. Methanol, Ethanol, Propanol, Butanol, Ethylenglykol sowie oxethylierten Monoalkoholen, wie Butylglykol, Isobutylglykol, Butyldiglykol gelöst.

### Beispiele

### Herstellung der Tri- und Tetracarbonsäureester

### Beispiel 1

### Umsetzung von Citronensäure mit Dibutylamintetraglykolether

In einem 250 ml Vierhalskolben mit Rührer, Thermometer, Stickstoffspülung sowie Destillationsbrücke wurden 29 g Citronensäure, 156 g Dibutylamintetraglycolether sowie 1,9 g p-Toluolsulfonsäure vermischt und auf 140-150°C erhitzt. Dann wurde so lange Wasser abdestilliert bis die Säurezahl max. 5 mg KOH/g betrug. Der resultierende Citronensäureester wies eine Verseifungszahl von 155,3 mg KOH/g auf.

### Beispiel 2

### Umsetzung von Trimellitsäureanhydrid mit Dibutylamintetraglykolether

In einem 250 ml Vierhalskolben mit Rührer, Thermometer, Stickstoffspülung sowie Destillationsbrücke wurden 32 g Trimellitsäureanhydrid, 165 g Dibutylamintetraglykolether sowie 1,9 g p-Toluolsulfonsäure vermischt und auf 140°C erhitzt, wobei sich ein homogene Reaktionsmischung bildete. Nun wurde innerhalb von 2 h auf 180-200°C erhitzt und so lange Wasser abdestilliert bis die Säurezahl max. 5 mg KOH/g betrug. Der resultierende Trimellitsäureester wies eine Verseifungszahl von 148,4 mg KOH/g auf.

### Beispiel 3

### Umsetzung von Pyromellitsäureanhydrid mit Dibutylamintetraglykolether

In einem 500 ml Vierhalskolben mit Rührer, Thermometer, Stickstoffspülung sowie Destillationsbrücke wurden 36 g Pyromellitsäureanhydrid, 220 g Dibutylamintetraglykolether sowie 1,9 g p-Toluolsulfonsäure vermischt und auf 140°C erhitzt, wobei sich ein homogene Reaktionsmischung bildete. Nun wurde innerhalb von 2 h auf 180-200°C erhitzt und so lange Wasser abdestilliert bis die Säurezahl max. 5 mg KOH/g betrug. Der resultierende Pyromellitsäureester wies eine Verseifungszahl von 159,8 mg KOH/g auf.

### Beispiel 4

Der Citronensäureester aus Beispiel 1 wurde mit Dimethylsulfat quaterniert.

### Beispiel 5

Der Trimellitsäureester aus Beispiel 2 wurde mit Dimethylsulfat quatemiert.

### Beispiel 6

Der Pyromellitsäureester aus Beispiel 3 wurde mit Dimethylsulfat quatemiert.

### Wirksamkeit der Polymere als Gashydratinhibitoren

Zur Untersuchung der inhibierenden Wirkung der Polyester wurde ein Stahlrührautoklav mit Temperatursteuerung, Druck- und Drehmomentaufnehmer mit 450 ml Innenvolumen benutzt. Für Untersuchungen zur kinetischen Inhibierung wurde der Autoklav mit destillierten Wasser und Gas im Volumenverhältnis 20:80 gefüllt, für Untersuchungen der Agglomeratinhibierung wurde zusätzlich Kondensat zugegeben. Abschließend wurden 90 bar Erdgas aufgedrückt.
Ausgehend von einer Anfangstemperatur von 17,5°C wurde innerhalb 2 h auf 2°C abgekühlt, dann 18 h bei 2°C gerührt und innerhalb von 2 h wieder auf 17,5°C aufgeheizt. Dabei wird zunächst eine Druckabnahme gemäß der thermischen Kompression des Gases beobachtet. Tritt während der Unterkühlzeit die Bildung von Gashydratkeimen auf, so verringert sich der gemessene Druck, wobei ein Anstieg des gemessenen Drehmoments und ein leichter Anstieg der Temperatur zu beobachten ist. Weiteres Wachstum und zunehmende Agglomeration der Hydratkeime führt ohne Inhibitor schnell zu einem weiteren Anstieg des Drehmomentes. Beim Aufwärmen des Gemisches zerfallen die Gashydrate, so dass man den Anfangszustand der Versuchsreihe erreicht.

Als Maß für die inhibierende Wirkung des Polymers wird die Zeit vom Erreichen der Minimaltemperatur von 2°C bis zur ersten Gasaufnahme (T_{ind}) bzw. die Zeit bis zum Anstieg des Drehmomentes (T_{agg}) benutzt. Lange Induktionszeiten bzw. Agglomerationszeiten weisen auf eine Wirkung als kinetischer Inhibitor hin. Das im Autoklaven gemessene Drehmoment dient dagegen als Größe für die Agglomeration der Hydratkristalle. Bei einem guten Antiagglomerant ist das Drehmoment, das sich nach Bildung von Gashydraten aufbaut, gegenüber dem Blindwert deutlich verringert. Im Idealfall bilden sich schneeartige, feine Hydratkristalle in der Kondensatphase, die sich nicht zusammenlagem und somit nicht zum Verstopfen der zum Gastransport und zur Gasförderung dienenden Installationen führen.

### Testergebnisse

### Zusammensetzung des verwendeten Erdgases:

Methan 87,6 %, Ethan 1,26 %, Propan 0,08 %, Butan 0,02 %, Kohlendioxid 0,35 %, Stickstoff 10.61%.
Unterkühlung unter die Gleichgewichtstemperatur der Hydratbildung bei 90 bar: 8,5°C.
Als Vergleichssubstanz wurde ein kommerziell erhältlicher Gashydratinhibitor auf Basis Polyvinylpyrrolidon verwendet. Die Dosierrate betrug bei allen Versuchen 5000 ppm, bezogen auf die Wasserphase.

**Tabelle 1**

| Ester aus Beispiel | T_{ind} (h) | T_{agg} (h) |
|---|---|---|
| Blindwert | 0 | 0 |
| 1 | 9,4 | 9,4 |
| 2 | 13,2 | 13,3 |
| 3 | 15,1 | 15,1 |
| Vergleich | 3,0 | 3,1 |

Wie aus den obigen Testresultaten zu erkennen ist, wirken die erfindungsgemäßen Tri- bzw. -tetraester als kinetische Hydratinhibitoren, und zeigen eine deutliche Verbesserung gegenüber dem Stand der Technik. Die Verbindungen sind biologisch abbaubar, wie im Folgenden gezeigt wird.

**Tabelle 2: (nach OECD 306)**

| Ester aus Beispiel | Biologischer Abbau in % |
|---|---|
| Vergleich | 6 |
| 1 | 62 |
| 2 | 40 |
| 3 | 37 |

Um die Wirkung als Agglomeratinhibitoren zu prüfen, wurde im oben verwendeten Testautoklaven Wasser und Testbenzin vorgelegt (20 % des Volumens im Verhältnis 1:2) und bezogen auf die Wasserphase 5000 ppm des jeweiligen Additivs zugegeben.
Bei einem Autoklavendruck von 90 bar und einer Rührgeschwindigkeit von 5000 U/min wurde die Temperatur von anfangs 17,5°C innerhalb 2 Stunden auf 2°C abgekühlt, dann 16 Stunden bei 2°C gerührt und wieder aufgewärmt. Dabei wurden die Agglomerationszeit bis zum Auftreten von Gashydrat-Agglomeraten und das dabei auftretende Drehmoment am Rührer gemessen, das ein Maß für die Agglomeration der Gashydrate ist. Als Vergleichssubstanz wurde ein kommerziell erhältlicher Anti-Agglomerant (quartäres Ammoniumsalz) herangezogen.

**Tabelle 3**

| Polyester aus Beispiel | T_{agg} (h) | Mₘₐₓ(Ncm) |
|---|---|---|
| Blindwert | 0,1 | 15,9 |
| 4 | 4,1 | 2,1 |
| 5 | 2,5 | 1,2 |
| 6 | 2,9 | 1,1 |
| Vergleich | 2,2 | 3,7 |

Wie aus diesen Beispielen zu ersehen ist, sind die gemessenen Drehmomente im Vergleich zum Blindwert trotz Gashydratbildung stark reduziert. Dies spricht für eine deutliche agglomeratinhibierende Wirkung der erfindungsgemäßen Produkte. Sämtliche Beispiele zeigen eine deutlich bessere Performance als der kommerziell erhältliche Anti-Agglomerant (Vergleich = Stand der Technik). Die Verbindungen sind biologisch abbaubar, wie im Folgenden gezeigt wird.

**Tabelle 4: (nach OECD 306)**

| Quarternierter Ester aus Beispiel | Biologischer Abbau in % |
|---|---|
| Vergleich | 6 |
| 4 | 35 |
| 5 | 27 |
| 6 | 25 |

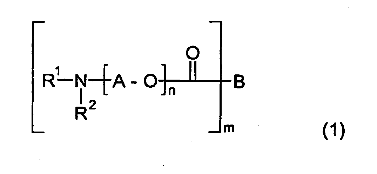

## Patentansprüche

1. Tri- und Tetracarbonsäureester der Formel (1) 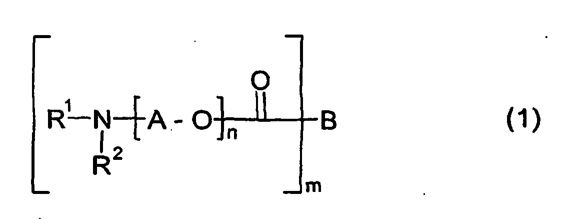 worin
R¹, R² unabhängig voneinander C₁- bis C₂₂-Alkyl, C₂- bis C₂₂-Alkenyl, C₆- bis C₃₀-Aryl- oder C₇- bis C₃₀-Alkylaryl, und,
A eine C₂- bis C₄-Alkylengruppe,
B eine beliebig substituierte organische Gruppe mit 3-100 Kohlenstoffatomen,
n eine Zahl von 1 bis 30
m 3 oder 4
bedeuten, wobei die Struktureinheiten -NR¹-R² teilweise oder vollständig quaterniert sein können.

2. Verbindungen nach Anspruch 1, wobei die Carbonsäure, aus der der Ester der Formel 1 hergestellt ist, Citronensäure, Pyrocitronensäure, Trimellitsäure, Pyromellitsäure, 1,2,3-Benzoltricarbonsäure, 1,3,5-Benzoltricarbonsäure, Butan-1,2,3,4-tetracarbonsäure, Trimellitsäureanhydrid, Pyromellitsäureanhydrid ist.

3. Verbindungen nach Anspruch 1 und/oder Anspruch 2, wobei A für eine Ethylen oder Propylengruppe steht.

4. Verbindungen nach einem oder mehreren der Ansprüche 1 bis 3, wobei die Struktureinheit -NR¹R² teilweise oder vollständig mit Methylchlorid, Benzylchlorid, Monochloressigsäure-Natriumsatz oder Dimethylsulfat quarterniert ist.

5. Verbindungen nach einem oder mehreren der Ansprüche 1 bis 3, wobei n für eine Zahl von 1 bis 6 steht.

6. Verwendung der Tri- und Tetracarbonsäureester gemäß einem oder mehreren der Ansprüche 1 bis 5 in Mengen von 0,01 bis 2 Gew.-% als Gashydratinhibitoren.

## Claims

1. A tri- and tetracarboxylic acid ester of the formula (1) 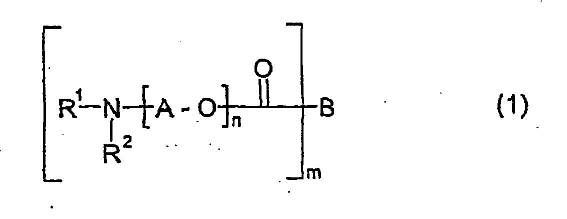 in which
R¹, R² independently of one another are C₁- to C₂₂-alkyl, C₂- to C₂₂-alkenyl, C₆- to C₃₀-aryl or C₇- to C₃₀-alkylaryl, and
A is a C₂- to C₄-alkylene group,
B is an arbitrarily substituted organic group having 3-100 carbon atoms,
n is a number from 1 to 30,
m is 3 or 4, it being possible for the structural units -NR¹-R² to be partly or completely quaternized.

2. A compound as claimed in claim 1, the carboxylic acid from which the ester of the formula 1 is prepared being citric acid, pyrocitric acid, trimellitic acid, pyromellitic acid, 1,2,3-benzenetricarboxylic acid, 1,3,5-benzenetricarboxylic acid, butane-1,2,3,4-tetracarboxylic acid, trimellitic anhydride, pyromellitic anhydride.

3. A compound as claimed in claim 1 and/or claim 2, A being an ethylene or propylene group.

4. A compound as claimed in one or more of claims 1 to 3, the structural unit -NR¹R² being partly or completely quaternized with methyl chloride, benzyl chloride, sodium monochloroacetate or dimethyl sulfate.

5. A compound as claimed in one or more of claims 1 to 3, n being a number from 1 to 6.

6. The use of the tri- and tetracarboxylic acid esters as claimed in one or more of claims 1 to 5 in amounts of from 0.01 to 2% by weight as gas hydrate inhibitors.

## Revendications

1. Esters d'acides tri- et tétracarboxyliques de formule (1) 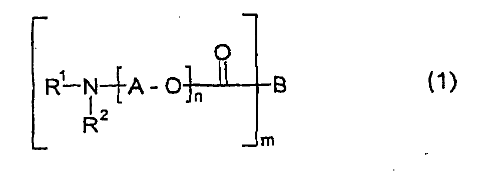 dans laquelle :
R¹ et R² représentent, indépendamment l'un de l'autre, un groupe alkyle en C₁ à C₂₂, un groupe alcényle en C₂ à C₂₂, un groupe aryle en C₆ à C₃₀ ou un groupe alkylaryle en C₇ à C₃₀,
A représente un groupe alkylène en C₂ à C₄,
B représente un groupe organique substitué de façon quelconque, renfermant de 3 à 100 atomes de carbone,
n représente un nombre de 1 à 30, et
m vaut 3 ou 4,
où les motifs structuraux -NR¹-R² peuvent être partiellement ou totalement quaternisés.

2. Composés selon la revendication 1, dans lesquels l'acide carboxylique à partir duquel l'ester de formule 1 est préparé est l'acide citrique, l'acide pyrocitrique, l'acide trimellitique, l'acide pyromellitique, l'acide 1,2,3-benzènetricarboxylique, l'acide 1,3,5-benzènetricarboxylique, l'acide butane-1,2,3,4-têtracarboxylique, l'anhydride trimellitique ou l'anhydride pyromellitique.

3. Composés selon la revendication 1 et/ou la revendication 2, dans lesquels A représente un groupe éthylène ou un groupe propylène.

4. Composés selon l'une ou plusieurs des revendications 1 à 3, dans lesquels le motif structural -NR¹-R² est partiellement ou totalement quaternisé avec du chlorure de méthyle, du chlorure de benzyle, du sel de sodium d'acide monochloroacétique ou du sulfate de diméthyle.

5. Composés selon l'une ou plusieurs des revendications 1 à 3, dans lesquels n représente un nombre de 1 à 6.

6. Utilisation des esters d'acides tri- et tétracarboxyliques, selon l'une ou plusieurs des revendications 1 à 5, en des quantités de 0,01 à 2 % en poids, en tant qu'inhibiteurs d'hydrates de gaz.
